# EUROPEAN PATENT SPECIFICATION

(11) **EP 0 592 582 B1**
(45) Date of publication and mention of the grant of the patent: **18.09.2002**
(21) Application number: 92915215.5
(22) Date of filing: 24.06.1992
(51) Int. Cl.: C12N 5/20, C12P 21/00, C07K 16/28, G01N 33/577, A61K 39/395, G01N 33/53

(54) **MONOCLONAL ANTIBODY TO LYMPHOCYTE-ASSOCIATED CELL SURFACE PROTEIN**
MONOKLONALER ANTIKÖRPER GEGEN LYMPHOZYTENASSOZIIERTES ZELLOBERFLÄCHENPROTEIN
ANTICORPS MONOCLONAL DIRIGE CONTRE UNE PROTEINE DE SURFACE CELLULAIRE ASSOCIEE A UN LYMPHOCYTE

(30) Priority: 25.06.1991 US 720602
(43) Date of publication of application: 20.04.1994
(73) Proprietor: DANA FARBER CANCER INSTITUTE, Boston Massachusetts 02115 (US)
(72) Inventor: TEDDER, Thomas, F., c/o Duke University Medical C., 353 Jones Bldg., Durham, NC 27710 (US); SPARTINI, Olivier, G., CH-1040 Echallens (Vaduz) (CH)
(74) Representative: Perry, Robert Edward
(86) International application number: US9205380
(87) International publication number: WO93000111

(56) References cited:
- EP-A- 0 386 906
- US-A- 5 098 833
- LEUKEMIA vol. 5, no. 4 , April 1991 , OXFORD, GB pages 300 - 308 O. SPERTINI ET AL. 'Regulation of leukocyte adhesion molecule-1 (TQ1, Leu-8) expression and shedding by normal and malignant cells.'
- THE JOURNAL OF IMMUNOLOGY vol. 144, no. 2 , 15 January 1990 , BALTIMORE MD, USA pages 532 - 540 T. TEDDER ET AL. 'Expression of the human leukocyte adhesion molecule, LAM1.'
- THE FASEB JOURNAL vol. 6, no. 5 , 28 February 1992 , BETHESDA MD, USA page A1889 L. FLAHERTY ET AL. 'A monoclonal antibody (MAb) to L-selectin does not prevent neutrophil (PMN) emigration into an inflamed peritoneal cavity.'
- THE JOURNAL OF CELL BIOLOGY vol. 114, no. 2 , July 1991 , NEW YORK NY, USA pages 351 - 358 G. KANSAS ET AL. 'Molecular mapping of functional domains of the leukocyte receptor for endothelium, LAM-1.'
- JOURNAL OF IMMUNOLOGY, Volume 128, No. 1, issued January 1982, E.L. REINHERZ et al., "Heterogeneity of Human T4+ Inducer T Cells Defined by a Monoclonal Antibody that Delineates Two Functional Subpopulations", pages 463-468.
- JOURNAL OF IMMUNOLOGY, Volume 129, No. 5, issued November 1982, P.A. GATENBY et al., "Dissection of Immunoregulatory Subpopulations of T Lymphocytes within the Helper and Suppressor Sublineages in Man", pages 197-200.
- PROC. NATL. ACAD. SCI. U.S.A., Volume 87, issued March 1990, T.K. KISHIMOTO et al., "Identification of a Human Peripheral Lymph Node Homing Receptor: A Rapidly Down-Regulated Adhesion Molecule", pages 2244-2248.
- JOURNAL OF CELL BIOLOGY, Volume 109, issued July 1989, B.R. BOWEN et al., "Characterization of a Human Homologue of the Murine Peripheral Lymph Node Homing Receptor", pages 421-427.
- JOURNAL OF EXPERIMENTAL MEDICINE, Volume 170, issued July 1989, T.F. TEDDER et al., "Isolation and Chromosomal Localization of cDNAs Encoding a Novel Human Lymphocyte Cell Surface Molecule, LAM-1", pages 123-133.
- JOURNAL OF CELL BIOLOGY, Volume 107, issued November 1988, N.W. WU et al., "Evolutionary Conservation of Tissue-Specific Lymphocyte-Endothelial Cell Recognition Mechanism Involved in Lymphocyte Homing", pages 1845-1851.

## Description

### Field of the Invention

This invention relates to a hybridoma cell line, to a monoclonal antibody produced by the hybridoma cell line, and to the therapeutic use of the antibody.

### Background of the Invention

An inducible endothelial-leukocyte adhesion molecule (ELAM-1) is expressed on the surface of cytokine-treated endothelial cells. This molecule is thought to be responsible for the accumulation of blood leukocytes at sites of inflammation, by mediating the adhesion of cells to the vascular lining (Bevilacqua *et al.*, Proc. Natl. Acad. Sci. USA 84:9238 (1987)). A granule membrane protein found in platelets and endothelial cells, termed GMP-140, has been cloned and is homologous with ELAM-1 (Johnson *et al*., Blood Suppl. 71:327a (1988)).

EP-A-0386906 discloses a human cDNA sequence encoding lymphocyte-associated cell surface protein LAM-1, which contains domains homologous with binding domains of animal lectins, growth factors and C3/C4 binding proteins.

Tedder *et al*., J. Immunol. 144(2):532-540 (1990), also discloses expression of LAM-1, and also the- generation of antibody-producing hybridomas. Spertini *et al*., Leukemia 5(4) :300-308 (1991), describes a further antibody to LAM-1, identified as anti-LAM1-3.

In EP-A-0386906, it is disclosed that antagonists to LAM-1 may be used to treat a human patient suffering from a lymphocyte-mobilizing condition.

Normal leukocytes have the ability to leave the circulation and localize in specific lymphoid organs or inflammatory sites through interactions between cell-surface receptors and ligands on endothelial cells [references 1-3, see Glossary]. The Leukocyte-Adhesion Molecule-1 (LAM-1) contains an amino-terminal, lectin-like domain which may interact with specific glyco-conjugates expressed on high endothelial venules (HEV) of peripheral LN (lymph nodes) and activated endothelium [3-5]. LAM-1 is expressed by human peripheral lymphocytes, neutrophils, eosinophils, monocytes and hematopoietic progenitor cells [5-8]. LAM-1 is expressed by the majority of circulating lymphocytes and memory T cells, but is lost following several days of mitogen stimulation [5,9,10]. In contrast, LAM-1 is shed from the cell surface within minutes of exposure of lymphocytes and neutrophils to PMA (phorbol 12-myristate 13-acetate) [5,6,11]. Both lymphocytes and neutrophils express a single LAM-1 protein product, but the molecular weight (Mr) of cell-surface LAM-1 on lymphocytes is 74,000 and that of neutrophils is 90,000-100,000 [6,9,12].

The specific adhesion of some tumor cells to the capillary endothelium and the existence of organ specific metastasis suggest that interactions between tumor cells and normal tissues influence tumor localization [13-15]. Although the molecules that mediate these events in malignant cells have not been completely described, many cell surface molecules involved in the adhesion and migration of normal leukocytes may be involved in the dissemination of hematopoietic malignant cells [1-3]. The mLHR has been implicated in the dissemination of lymphomas [14-16], and a calcium-dependent phosphomannosyl-binding site on human malignant lymphoblastoid cell lines mediates peripheral LN HEV binding [17].

The LAM-1 molecule is a member of a new family of cellular adhesion/homing molecules that contain a lectin-like domain at their amino-terminal end followed by an epidermal growth factor-like domain and short consensus repeat units like those found in C3/C4 binding proteins (consensus repeat units characteristic of complement-binding proteins). **J. Exp. Med.**, 170: 123-133 (1989) [4] and EP-A-0 386 906 report the isolation and chromosomal localization of cDNAs encoding the novel cell surface molecule LAM-1. In **Eur. J. Immunol.**, 20: 1351-1355 (1990), T.F. Tedder et al. reported that human antigen-specific memory T cells express the LAM-1 necessary for lymphocyte recirculation. In **J. Biological Chemistry**, 265: 7760-7677 (1990), Ord et al. (under the auspices of T.F. Tedder) reported the structure of the gene encoding the LAM-1 of lymphocytes and neutrophils. In **J. Immunology**, 144: 532-542 (1990) [5], T.F. Tedder et al. described two monoclonal antibodies, LAM1-1 and LAM1-2, that react with LAM-1. [Note: As used herein, LAM-1 refers to the leukocyte-adhesion molecule-1 itself and "LAM1-X" refers to an antibody x which binds to an epitope of LAM-1.}

The monoclonal antibodies LAM1-1 and LAM1-2 were found to be reactive with the majority of blood lymphocytes, NK (Natural Killer) cells, neutrophils, monocytes and hematopoeitic progenitor cells. Binding of LAM-1 may participate in the process of leukocyte extravasation into lymphoid organs or sites of acute inflammation with subsequent loss of LAM-1 from the cell surface. LAM-1 is also recognized by the TQ1 and Leu-8 monoclonal antibodies that have been previously identified.

The loss of LAM-1 expression after leukocyte activation in vivo, with the concomitant increase in expression of CD2, CD18, CDlla or CD11b may result in significant and dramatic increases in migration and ability to recognize endothelial cell surfaces. Of significance is the fact that patients with AIDS have diminished expression of LAM-1 on their T and B cells. This may also occur in other immunological syndromes. Therefore, alterations in LAM-1 expression by neutrophils are significant because the mLHR is involved in neutrophil migration into sites of acute inflammation. LAM-1, in conjunction with other selectins and receptors, is involved in the extravasation of most leukocytes. The expression of LAM-1 by different leukocytes sub-populations thus plays a key role in determining the characteristics and magnitude of local immune responses [5].

There is growing evidence in mouse and man that the binding of lymphocytes to HEV of peripheral LN and the migration of normal leukocytes from blood into inflammatory lesions are controlled by several adhesion molecules whose expression is coordinately regulated [5,6,33,43]. It is likely that similar mechanisms will contribute to the spread of leukemias and lymphomas. In one murine study, the expression of functional receptors for HEV was shown to control the hematogenous dissemination of malignant lymphocyte populations to HEV-bearing organs [16]. Lymphomas that bound well to HEV disseminated weakly via the blood, ultimately involving all LN groups symmetrically. In contrast, gross involvement of LN by non-binding lymphomas was limited to nodes draining localized tumors which formed at the site of injection. These results suggested that the expression of functional receptors for HEV either controls the hematogenous dissemination of malignant lymphocyte populations to HEV-bearing organs, or is co-regulated with factors that determine metastatic behaviour [16]. In humans, the expression of functional HEV-binding molecules, such as LAM-1, on CLL and low-grade lymphoma cells may also contribute to the wide-spread dissemination of these malignant cells to LN, as occurs with normal lymphocytes.

### Summary of the Invention

According to a first aspect of the present invention, a hydridoma cell line has American Type Culture Collection Deposit Number HB 10771.

According to a second aspect of the present invention, an anti-LAM1-3 monoclonal antibody is obtainable from the hydridoma cell line defined above.

According to a further aspect of the present invention, a chimeric antibody comprises the variable region of the anti-LAM1-3 antibody defined above and a constant region derived from a human monoclonal antibody.

An antibody of the invention may be used in therapy, specifically for use in blocking adhesion, migration and infiltration into tissues of cells expressing LAM-1.

Further, anti-LAM1-3 is useful in radioisotope or immunofluorescent assays for the detection of LAM-1, e.g. for identifying species which have or do not have LAM-1. The antibody is further useful for separating cells expressing LAM-1 from cells not expressing LAM-1 or *vice versa.* Furthermore, this monoclonal antibody also completely blocks leukocyte attachment to HEV or endothelium.

Neutrophil-mediated inflammation is involved in a number of human clinical manifestations, including the adult respiratory distress syndrome, multi-organ failure and reperfusion injury. One way of inhibiting this type of inflammatory response would be to block competitively the adhesive interactions between neutrophils and the endothelium adjacent to the inflamed region. Anti-LAM1-3 reacts with LAM-1 on many animal species, but does not bind the mLHR. Anti-LAM1-3 blocks completely lymphocytic traffic to lymph nodes and extravasation of neutrophils from blood to inflammatory sites. The administration of soluble forms of anti-LAM1-3 could be clinically effective for the inhibition of neutrophil-mediated inflammation. Anti-LAM1-3 also blocks lymphocyte adhesion to human HEV and activated endothelium. Therefore, it is likely that the use of anti-LAM1-3 will block lymphocyte entry into sites of inflammation or tissue injury. Such activity will be useful for preventing kidney or other organ transplant rejection which is mediated by lymphocytes.

It is also within the scope of the invention to prepare chimerized monoclonal antibodies from the mouse antibodies. Antibodies are Y-shaped molecules consisting of two long "heavy" chains which define the stem and arms of the Y and two short "light" chains which are attached to the outside of the arms. The amino-terminal ends of the arms of the antibody molecule contain the variable regions of the antibody. The variable regions are specific for a particular antigen. The stem of the molecule is the "constant" region which ends in a carboxylate function (COO) and remains the same from molecule to molecule in antibodies of the same isotype in the same species.

The constant region of the mouse antibody has been found to be the primary source human immune reactions to mouse monoclonal antibodies. Using standard genetic engineering techniques, mouse variable regions have been fused to human constant regions to generate "chimeric" (from chimera or chimaera, a monster of Greek mythology which had a lion's head, a goat's body and a serpent's tail) antibodies. These chimeric antibodies thus possess regions of different genetic origin and have been found to have a lower tendency to produce allergic reactions.

The hybridoma cell line was produced by the fusion of NS-1 myeloma cells with spleen cells obtained from mice immunized with LAM-1 cDNA-transfected 300.19 cells (a mouse pre-B cell line). The hybridoma cell line produces a monoclonal antibody reactive with human, monkey, cow, rabbit, sheep, dog, cat, pig and goat leukocyte adhesion molecule-1, LAM-1. The monoclonal antibody produced by the cell line of the claimed invention, identified as anti-LAM1-3, may be clinically useful in blocking leukocyte entry into sites of inflammation or tissue injury.

### BRIEF DESCRIPTION OF THE DRAWINGS

Fig. 1 depicts the immunoprecipitation of LAM-1 shed from a cell surface with anti-LAM1 antibodies or a control immunoprecipitation with an unreactive isotype-matched antibody with subsequent sodium dodecyl sulfate-polyacrylamide gel electrophoresis;
Fig. 2 depicts the percentage and reactivity of malignant cells being LAM-1 positive from patients having various forms of hematopoietic malignancies;
Fig. 3 depicts the immunoprecipitation of LAM-1 from the surface of iodinated CLL cells using anti LAM1-1 antibodies or an unreactive isotype-matched control with subsequent sodium dodecyl sulfate-polyacrylamide gel electrophoresis under reduced and non-reduced conditions; and
Fig. 4 depicts indirect immunofluorescence results obtained with the anti-LAM1-3 antibody and PPME-FITC.

### STATEMENT OF DEPOSIT

The hybrid cell line which produces the anti-LAM-1 monoclonal antibody anti-LAM1-3 embodying this invention was deposited with the American Type Culture Collection, 12301 Parklawn Drive, Rockville, Maryland 20852 on June 12, 1991 and is assigned A.T.C.C. Deposit No. HB 10771.

### Glossary

### References

1. L.M. Stoolman et al.. Cell 56: 907-910 (1989).
2. A. Duijvestijn et al., Immunol. Today 10: 23-28 (1989).
3. E.L. Berg et al., Immunol. Rev. 108: 5-18 (1989).
4. T.F. Tedder et al., J. Exp. Med. 170: 123-133 (1989).
5. T.F. Tedder et al., J. Immunol. 144: 532-540 (1989).
6. J.D. Griffith et al., J. Immunol. 145:576-584 (1990).
7. G,S. Kansas, J. Immunol. 134: 3003-3006 (1985).
8. E.L. Reinherz et al., J. Immunol. 1228: 463-468 (1982).
9. T.F. Tedder et al., Eur. J. Immunol. 20: 1351-1355 (1990).
10. M.E. Kanop et al., J. Immunol. 140: 3701-3706 (1988).
11. T.K. Kishimoto et al., Proc. Natl. Acad. Sci. 87: 2244-2248 (1990).
12. D.C. Ord et al., J. Biol. Chem. 14: 7760-7767 (1990).
13. L. Weiss et al., FASB J. 2: 12-21 (1988).
14. B.T. Sher et al., Adv. Can. Res. 51: 361-389 (1988).
15. G.E. Rice et al., Science 246: 12303-1306 (1989).
16. R.F. Bargatze et al., J. Exp. Med. 166:1125-1131 (1987).
17. L.M. Stoolman et al., J. Clin. Invest. 84: 1196-1205 (1989).
18. T.F. Tedder et al., Eur. J. Immunol. 16: 1539-1543 (1986).
19. F. Sanchez-Madrid et al., Proc. Natl. Acad. Sci. 79: 7489-7483 (1982).
20. R. Rothlein et al., J. Immunol. 137: 1270-1274 (1986).
21. G.S. Kansas et al., J. Immunol. 142: 3050-3057 (1989).
22. N. Dana et al., J. Immunol. 137: 3259-3263 (1986).
23. T.A. Yednock et al., J. Cell Biol. (1987).
24. T.A. Yednock et al., J. Cell Biol. 104: 725-731 (1987).
25. H.B. Stamper et al., J. Exp. Med. 144: 828-833 (1976)
26. E.C. Butcher et al., J. Immunol. 134: 2829 (1979).
27. T.F. Tedder et al., Molecular Immunol. 25: 1321-1330 (1988).
28. S. Jalkanen et al., Eur. J. Immunol. 16: 1195-1202 (1986).
29. S. Jalkanen et al., J. Cell Biol. 105: 893-990 (1987).
30. T.F. Tedder et al., J. Immunol. 134: 2989-2994 (1985).
31. H. Hidaka et al., Biochemistry 23: 5036-5040 (1984).
32. T. Tamaoki et al., Biochem. Biophys. Res. Commun. 135: 397-402 (1986).
33. T.K. Kishimoto et al., Science 245: 1238-1241 (1989).
34. T.M. Jung et al., J. Immunol. 144: 130-3136 (1990).
35. S.A. Michie et al., Am. J. Clin. Pathol. 88: 486-490 (1987).
36. A. Carbone et al., J. Pathol. 154: 133-140 (1988).
37. J.G. Strickler et al., Hum. Pathol. 19: 550-554 (1988).
38. G.S. Kansas et al., J. Immunol. 142: 3058-3062 (1989).
39. K. Miyake et al., J. Exp. Med. 172: 69-75 (1990).
40. S.T. Pals et al., Blood, 73: 885-888 (1989).
41. S. Aizawa et al., Proc. Natl. Acad. Sci. 85: 3180-3183 (1988).
42. L.M. Stoolman et al., J. Cell Biol. 99: 1535-1540 (1984).
43. M.A. Jutila et al., J. Immunol. 143: 3318-3324 (1989).

### Definitions and Abbreviations

- Selectins =: a recently described family of cellular adhesion/homing receptor molecules identified by cDNA cloning. Members of this family include the leukocyte adhesion molecule-1 (LAM-1) which is the human homolog of the mouse lymphocyte homing receptor (mLHR), the human granule-membrane protein (GMP-140, PADGEM, CD62) which is expressed on activated platelets and endothelial cells, and the human endothelial leukocyte adhesion molecule-1 (ELAM-1) expressed on activated endothelial cells. The name "selectins" has been suggested for this family because of the presence of the lectin domain and their role in selective cell trafficking.
- LN =: lymph node
- PMA =: phorbol 12-myristate 13-acetate
- PKC =: protein kinease C
- LAM-1 =: leukocyte adhesion molecule-1
- CLL =: chronic lymphocytic leukemia
- NHL =: non-Hodgkin's lymphoma
- PPME =: xpoly-phosphomonoester from the yeast

### HANSENULA

### cell wall

- AML =: acute myelogenous leukemia
- CML =: chronic myelogenous leukemia
- PBMC =: peripheral blood mononuclear cells
- BM =: bone marrow
- RPMI Medium =: commercial product available from Gibco, Walkersville, Maryland.
- FSC =: follicular small cleaved cell lymphoma
- CSF =: colony stimulating factor
- DSC =: diffuse small cleaved cell lymphoma
- FITC =: fluorescein isothiocyanate
- LPS =: lipopolysaccharide
- kb =: kilobase

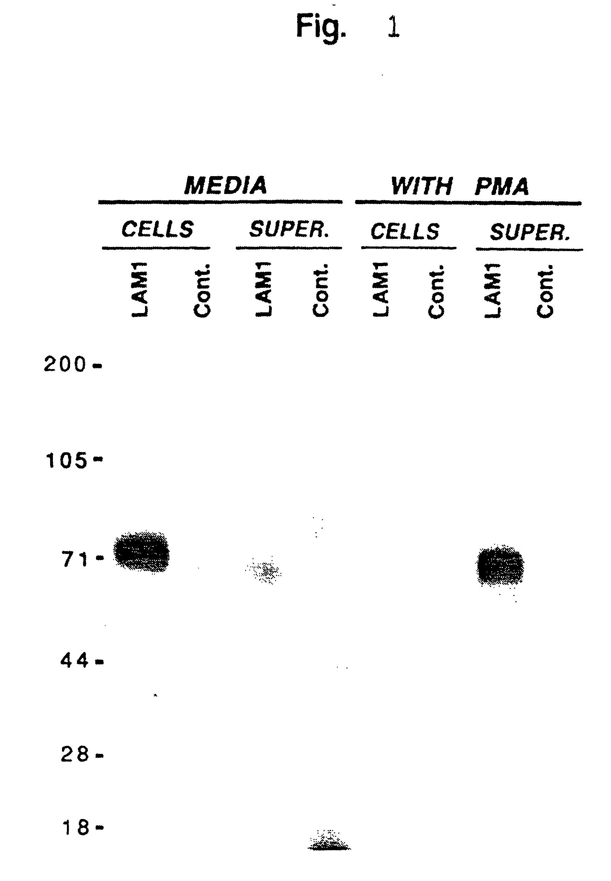

Blood lymphocytes were isolated and incubated for 20 minutes at 4°C with an antibody (see Table 1); anti-CD3 was crosslinked as shown by Spertini et al., Nature 349:691-694 (1991); see Fig. 1. After one wash, the cells were incubated with ¹²⁵I-labelled PPME (90.36 µg/ml, 2.2x10⁵ c.p.m. per sample) at 4°C for 30 minutes. Anti-LAM1-3 was added 1 minute before the addition of the test antibody and during all incubations. The calcium-independent binding of [¹²⁵I]PPME was assessed in the presence of 5 mM EDTA. Cells were washed, resuspended in PBS-BSA and layered on a 750 µl cushion of 75% (v/v) calf serum. The cell pellet was isolated, and bound [¹²⁵I]PPME was assessed by y (gamma) counting. The data shown in Table 1 are representative of those obtained in three experiments. Fluorescein-labelled PPME was iodinated by standard methods, the specific activity (2x10⁴ c.p.m./ng) determined by self-displacement curve analysis, and the maximum binding capacity was 20%.

HEV binding was assessed using 12-µm freshly cut, frozen rat lymph node sections. The number of lymphocytes bound to HEV was counted on coded slides. The results are shown in Table 1, wherein the values are means ± standard deviation (s.d.) of four experiments, and the differences between control antibody-treated cells and anti-CD2 and anti-CD3 treated cells were significant.

**Table 1**

| Binding of [¹²⁵I]PPME (c.p.m. ± s.d.) | | | | Cells Bound/HEV (± s.d.) | |
|---|---|---|---|---|---|
| Stimulus | Medium | anti-LAM-3 | EDTA | Medium | anti-LAM1-3 |
| Medium | 4847±372 | 1160±104 | 578 | 1.4±0.2 | 0.2±0.2 |
| anti-LAM1-10 | 6868±571 | 2103±905 | 2030 | 1.5±0.8 | 0.2±0.2 |
| anti-CD2 | 17886±419 | 2863±689 | 965 | 2.4±0.9¹ | 0.2±0.2 |
| anti-CD3 | 19718±1294 | 1211±618 | 303 | 2.7±1.0² | 0.2±0.2 |

| | | | | | |
|---|---|---|---|---|---|
| 1. P<0.01 using the paired Student's t-test. | | | | | |
| 2. P<0.05 using the paired Student's t-test. | | | | | |

The level of HEV binding was also proportional to the quantity of LAM-1 expressed, and LAM-1 negative cells were unable to bind HEV and PPME. LAM-1 was also shed from the surface of CLL cells following PMA exposure. However, the signalling pathway for shedding may be less active in some CLL cells since the time-course of LAM-1 shedding was slower than in normal lymphocytes. Malignant cells, therefore, express function LAM-1 receptors that are indistinguishable from their normal counterparts on normal cells and the expression of LAM-1 by CLL cells correlated with the high tendency of these cells to localize into peripheral LN.

### Materials and methods

### Cell Samples

Peripheral blood mononuclear cells (PBMC) were isolated by Ficoll-Hypaque density gradient centrifugation of blood, bone marrow (BM) samples and single cell suspensions of LN. Cells were obtained by protocols approved by the Human Protection Committee of the Dana-Farber Cancer Institute. Tumor type was classified according to conventional morphological, cytological and immunophenotype criteria. Tumor cell lineage was determined by analysis of antigens (Ag) including surface and cytoplasmic immunoglobulin (Ig), HLA-DR Ag, CD1, CD2, CD3, CD4, CD5, CD6, CD8, CD9, CD10, CD11b, CD13, CD14, CD19, CD20 and CD33. Cells were examined immediately after isolation or were immediately cryopreserved and kept frozen in liquid nitrogen until used. The frequency of malignant cells was always greater than 90% in every sample examined.

### Antibodies.

The anti-LAM-1 monoclonal antibodies anti-LAM1-1 and anti-LAM1-2 and the monoclonal antibody anti-TQ1 have been previously described [5,8]. The anti-LAM1-3 antibody (IgG1) of the claimed invention was generated by the fusion of NS-1 myeloma cells with spleen cells from Balb/c mice that were repeatedly immunized with cells of the mouse pre-B cell line 300.19 transfected with a LAM-1 cDNA as described [5]. The antibodies used in these studies included: 2F12 (CD11a) and 10F12 (CD18) [18] which were gifts from J. Ritz (Dana-Farber Cancer Inst., Boston MA); TS2/9 (CD58, anti-LFA-3) [19] and RR 1/1 (CD54, anti-ICAM-1) [20] which were gifts from T.A. Springer (Center for Blood Research, Boston, MA); 515 (CD44) [21] a gift from G.S. Kansas (Dana-Farber Cancer Inst.); and 904 (CD11b) [22].

### Immunofluorescence analysis.

Indirect immunofluorescence analysis was performed on viable cells isolated by Ficoll-Hypaque density gradient centrifugation. The expression of LAM-1, CDlla, CD11b, the β subunit of CD11 complex (CD18), CD44, CD54, and CD58 was examined by indirect immunofluorescence with flow cytometry analysis (Coulter Epics C, Coulter Electronics, Hialeah FL). Isotype-matched murine antibodies that were unreactive with human leukocytes were used as negative controls. Cells were incubated with each monoclonal antibody for 20 minutes on ice, washed, and treated with FITC-conjugated goat anti-mouse Ig reagents (Southern Biotechnology Associates, Birmingham, AL).

The ability of normal lymphocytes and B-CLL cells to bind the fluorescein derivative of PPME (fl-PPME) (a gift of S.D. Rosen, University of California, San Francisco, CA) was assessed by incubating cells for 30 minutes on ice with 100 µl of fl-PPME at 30 µg/ml in phosphate buffered saline (PBS). After washing twice, the binding of fl-PPME was examined by flow cytometry analysis as described [23,24].

### HEV binding assay.

The in vitro HEV binding assay was performed using frozen tissue sections of human or rat peripheral LN using the methods of Stamper and Woodruff [25] and Butcher, et al. [26] as described [5]. Blocking of cell binding using the anti-LAM-1 monoclonal antibodies was carried out using freshly cut-frozen rat lymph node sections and the antibodies were used as ascites fluid at dilutions of 1:100.

### Immunoprecipitation analysis

Cells were washed twice, resuspended in RPMI 1640 medium (Sigma, St. Louis, MO) at a concentration of 30 x 10⁶ cells/ml and treated for 40 minutes at room temperature with neuraminidase (0.1 U/ml, Calbiochem, La Jolla, CA) and then labelled by lactoperoxidase-catalyzed iodination. After washing, the cells were lysed in buffer containing 1% (v/v) NP-40 as described [27]. Cell lysates were precleared for 2 hours using 3 µl of murine ascites fluid (isotype matched antibody) and 25 µl of a 50% (v/v) suspension of Gammabind-G Agarose (Genex, Gaithersburg, MD). Cell lysates were precleared again overnight. Half of the precleared lysate was then incubated with 3 µl of anti-TQ1 ascites fluid, 3 µl of anti-LAM1-1 ascites fluid, and 50 µl of Gammabind-G with constant rotation at 4°C for 18 hours. The other half of the lysate was treated similarly using 3 µl of isotype-matched murine ascites fluid. Immunoprecipitates were washed and analyzed by SDS-PAGE. Molecular weights (Mr) were determined using standard molecular weight markers (BRL, Bethesda, MD).

In experiments designed to study LAM-1 shedding, LAM-1 was immunoprecipitated as described above from the supernatant fluid and the pellet of PBMC that had been cultured for 60 minutes at 37°C in RPMI 1640 medium alone or in RPMI medium containing PMA (100 ng/ml, Sigma, St. Louis, MO). In addition, expression of LAM-1 was assessed after incubation of the cells with PMA (10 nM for 30 minutes) following the prior culture of the cells with sodium azide (Sigma) or the protein kinase inhibitors, 1-(5-Isoquinolinyl-sulfonyl)-2-methylpiperazine (H-7, Calbiochem) and staurosporine (Sigma) for 30 minutes at 37°C.

### Discussion of Figures 1-4.

### Figure 1.

LAM-1 is shed from the cell surface into the culture medium. PBMC were surface iodinated and cultured for 60 minutes in medium or medium containing 100 ng/ml PMA. The supernatant fluid and cells were harvested and immunoprecipitated with anti-LAM-1 antibodies or an unreactive isotype matched control antibody (Cont.). Immunoprecipitated materials were electrophoresed on a 7.5% SDS acrylamide gel under reducing conditions followed by autoradiography. The migration of known molecular weight standards is shown in kilo-Daltons (kDa).

### Figure 2.

The frequency of LAM-1 expression by malignant cells. Cells from 118 patients with various forms of hematopoietic malignancies were examined for surface LAM-1 expression using the anti-LAM1-1 monoclonal antibody in indirect immunofluorescence assays with flow cytometry analysis. In each instance, the background staining for each sample was determined using an unreactive isotype-matched monoclonal antibody and the level of background staining (usually less than 5%) was subtracted from the values shown. The horizontal bars represent the mean frequency of reactive cells. [Abbreviations: Pre-B, pre-B acute lymphoblastic leukemia; T-ALL, T cell acute lymphoblastic leukemia; B-CLL, B type chronic lymphocytic leukemia; FSC, follicular small cleaved cell lymphoma; DSC, diffuse small cleaved cell lymphoma; DLC, diffuse large cell lymphoma; Burk., Burkitt's type lymphoma; M.M., multiple myeloma; AML, acute myelogenous leukemia; CML, chronic myelogenous leukemia]. The relative fluorescence staining intensity of the malignant cells is indicated where the positive population could be identified as a distinguishable peak from background fluorescence staining: ±, where a shoulder of positively stained cells was evident, +, where a separate peak of positive cells was identified with weak fluorescence; ++, a definite separate peak of fluorescence positive cells of moderate fluorescence; +++, a peak of fluorescence positive cells of the same intensity as normal blood lymphocytes. The tissue source of all malignant cells is also indicated.

### Figure 3.

Analysis of LAM-1 immunoprecipitated from B-CLL cells. Detergent lysates of surface iodinated cells (45 x 10⁶) were immunoprecipitated with the anti-TQ1 and anti- LAM1-1 antibodies (LAM-1) or an unreactive isotype-matched antibody control (Cont.). Immunoprecipitated materials were divided and analyzed under non-reducing and reducing conditions on a 12% SDS polyacrylamide gel followed by autoradiography. Molecular weights (kDa) were determined by the migration of known protein standards.

### Figure 4.

Normal human lymphocytes and CLL cells are capable of binding PPME through LAM-1. Cells were examined for LAM-1 expression by indirect immunofluorescence analysis after treatment with the anti-LAM1-3 monoclonal antibody (dark line) or with an unreactive isotype matched antibody (thin line). Cells were also reacted with FITC-conjugated PPME after treatment with the anti-LAM1-3 antibody (thin line) or an unreactive control antibody (dark line). The fluorescence intensity of cell staining was analyzed by flow cytometry.

### Results

### LAM-1 is released from the cell surface following PMA exposure.

Immunoprecipitation experiments were carried out to determine the fate of LAM-1 after modulation from the surface of PBMC exposed to PMA [5]. Surface iodinated cells were cultured for 60 minutes in RPMI medium alone or medium containing PMA. After culture, the supernatant fluid and cells were separated, and the cells were lysed with detergent. The cell lysate and supernatant fluid were immunoprecipitated with a combination of anti-LAM1-1 and anti-TQ1 antibodies that bind to different epitopes of LAM-1 [5], and together are more efficient for immunoprecipitation. The cells were also treated with neuraminidase prior to surface iodination since LAM-1 may be more readily immunoprecipitated after the removal of sialic acid residues. Treatment of cells with PMA resulted in a dramatic loss of immunoprecipitable LAM-1 from the cell surface with a concomitant increase in the level of LAM-1 precipitated from the supernatant fluid. Incubation of cells in medium without PMA also resulted in a small amount of LAM-1 being found in the supernatant fluid (Fig. 1). The molecular weight of LAM-1 precipitated from the supernatant fluid was slightly smaller (by about 5 kDa) than the species of LAM-1 found on the cell surface. Interestingly, the residual LAM-1 found on the cell surface of PMA-treated cells was most similar in molecular weight to that of the LAM-1 found in the supernatant fluid. The quantitative recovery of labeled LAM-1 from the supernatant fluid, in comparison to the amount immunoprecipitated from solubilized cells, demonstrates that a major portion of LAM-1 is shed from the cell surface and not internalized following PMA exposure.

### Expression of adhesion molecules by malignant leukocytes.

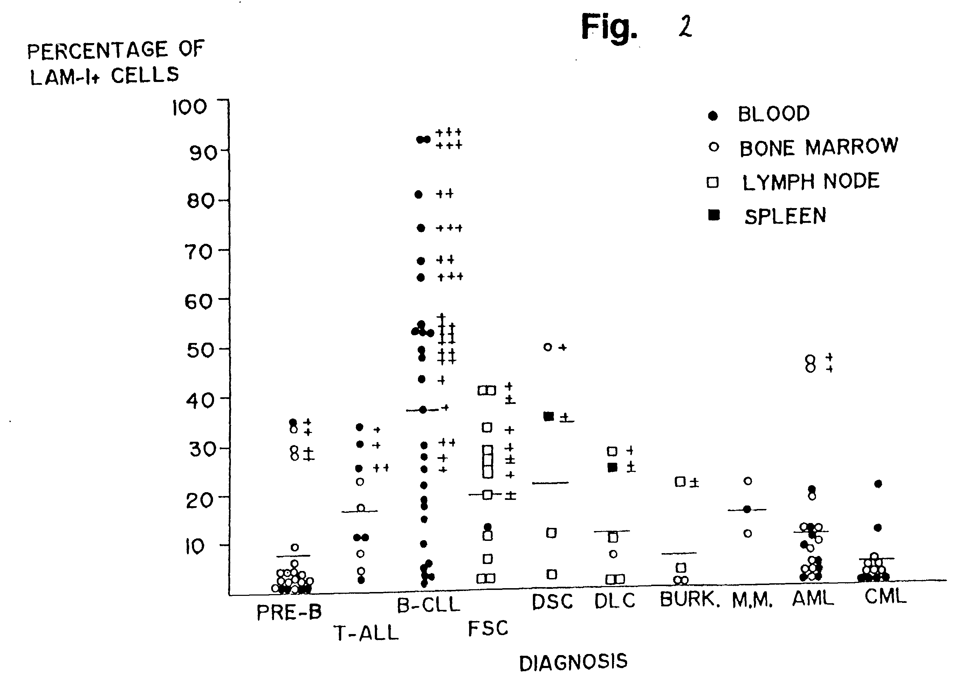

The expression of LAM-1 and other cell surface molecules known to be involved in lymphocyte adhesion and migration was examined on malignant leukocytes from 118 patients by indirect immunofluorescence analysis. LAM-1 expression was most frequently demonstrated on CLL cells and among lymphomas classified as follicular (FSC) and diffuse small cleaved cell lymphoma (DSC). On the other hand, most acute myeloblastic leukemia (AML), acute lymphoblastic leukemia (ALL), chronic myelocytic leukemia (CML), diffuse large cell lymphomas (DLC), Burkitt's lymphomas and multiple myelomas were LAM-1 negative. The level of cell surface LAM-1 expression was highest on CLL cells; and in 11 of 16 cases that expressed LAM-1, more than 50% of cells were LAM-1+ (FIG. 2). In general, the fluorescence intensity level of LAM-1 staining correlated with the frequency of LAM-1+ cells such that most malignant cell populations with less than 25% positive cells failed to express LAM-1 at easily detectable levels.

CLL cells rarely expressed adhesion molecules other than LAM-1, with the exception of CD44, which has also been associated with lymphocyte homing [28,29]. More than 90% of the cell samples were CD44+, consistent with its ubiquitous distribution on normal hematopoietic cells. Ninety-three percent of the leukemias and 84% of the B-NHL were positive for this antigen and generally greater than 60% of the cells were CD44+. In general, the expression of CDlla, CD11b, CD18, CD54, and CD58, was more heterogeneous .

Incubating normal blood lymphocytes for about 8-16 hours at 4°C can result in complete loss of LAM-1 from the cell surface [30]. However, the cryopreservation of PBMC, LAM-1+ lymphoblastoid cell lines and freshly isolated LAM-1+ malignant cells did not appreciably alter the qualitative expression of LAM-1. A minor quantitative decrease in LAM-1 expression was observed that could be reversed by culturing the cells for about 8-16 hours in RPMI 1640 medium with 10% FCS (fetal calf serum). Consequently, 37 of the samples of malignant cells were re-examined following the above culture. This treatment, however, did not result in the appearance of LAM-1 on the cell surfaces or a significant increase in the frequency of LAM-1 expression in any case.

### Structure of LAM-1 expressed by malignant cells.

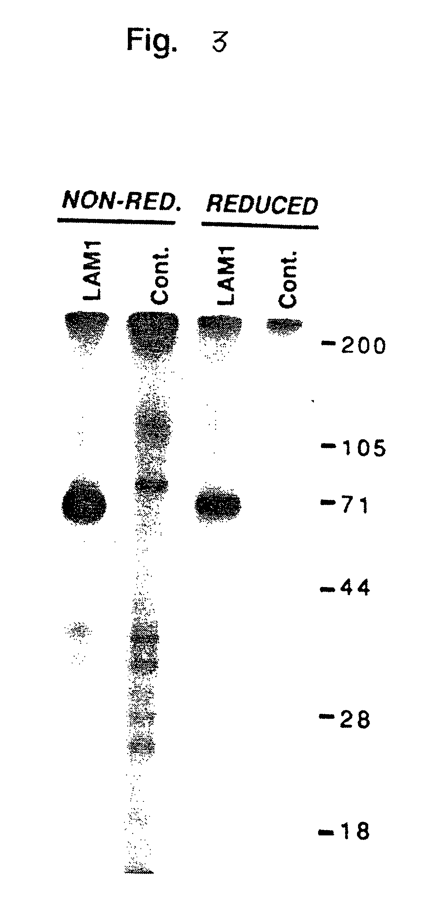

Anti-LAM-1 antibodies were used to immunoprecipitate LAM-1 from CLL cells. LAM-1 migrated with a Mr of 68,000 under non-reducing conditions and at 73,000 after reduction (FIG. 3), similar to LAM-1 immunoprecipitated from normal lymphocytes (FIG. 1). Therefore, it appears that normal and malignant lymphocytes express the same cell-surface LAM-1 protein.

### LAM-1 receptor function.

The relationship between LAM-1 expression and the ability of cells to bind to human peripheral LN HEV was examined using cells from normal circulating blood, three LAM-1 positive CLLs and one LAM-1 negative CLL. Cells were assessed for their ability to bind HEV of human peripheral LN using the frozen section assay of Stamper and Woodruff [25]. The LAM-1+ cells bound to HEV at levels which corresponded to the amount of LAM-1 expressed on their cell surface, while the LAM-1 CLL cells did not bind. In contrast, CD44 expression was quite high on all of the cell samples examined and did not correlate with HEV adhesion. Additional studies examined the ability of anti-LAM-1 monoclonal antibody to block HEV binding. The new antibody, anti-LAM1-3, was able to specifically block 92 to 95% of normal lymphocyte and LAM-1+ CLL cell binding to rat peripheral LN HEV. In contrast, the binding of a different antibody, anti-LAM1-10, reactive with a different epitope of LAM-1, had no detectable effect on HEV binding. (See Table 1). Therefore, the levels of LAM-1 expression correlated with the ability of cells to bind HEV and antibodies reactive with LAM-1 specifically blocked binding.

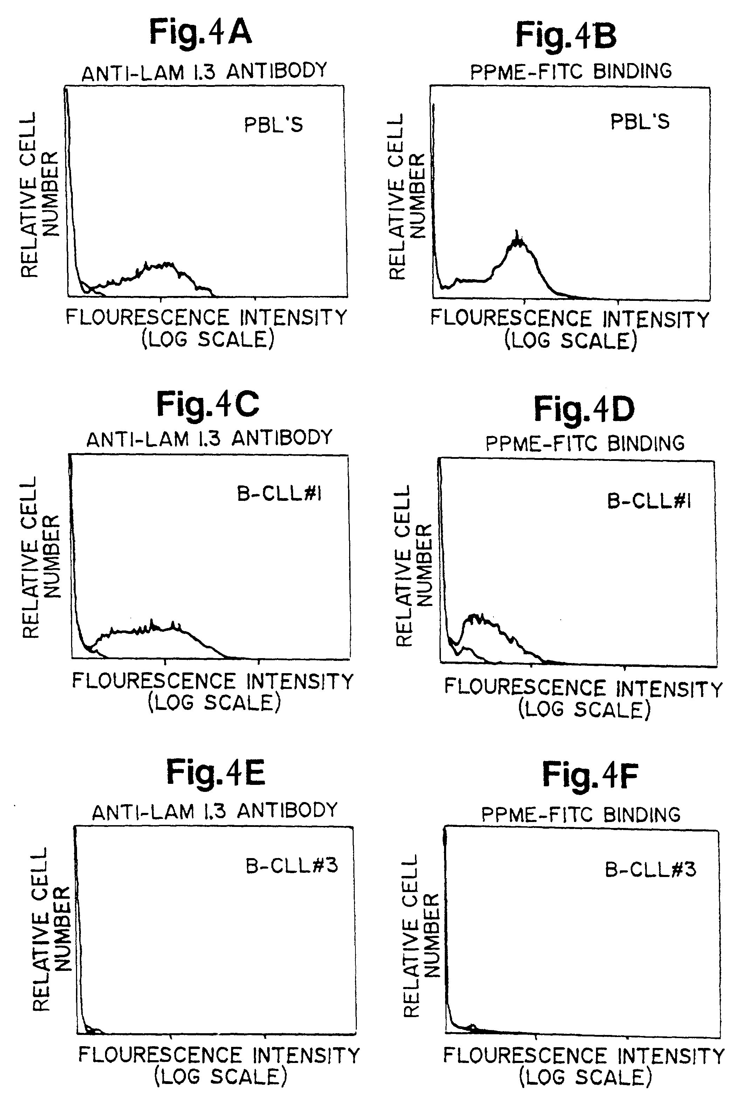

The ligand for the mLHR is mimicked by the mannose 6 phosphate-rich polysaccharide PPME [24]. Therefore, the ability of normal human lymphocytes and CLL cells to bind fluoresceinated PPME was examined to further characterize the functional capacity of human LAM-1. Both normal blood lymphocytes and LAM-1+ CLL cells were able to bind PPME, while LAM-1 CLL cells did not bind PPME (FIG. 4). The specificity of PPME binding to LAM-1 was verified by the ability of anti-LAM1-3 antibody to completely block PPME binding to the cells (FIG. 4).

Table 2, below, lists a member of properties of the monoclonal antibodies anti-LAM1-1, -2 and -3, including the isotype and the differences in staining intensity. These properties are not necesarily indicative of differences in epitope recognition.

Functional studies of the antibodies are given in lines 4-6. These results demonstrate that different parts of the LAM-1 molecule are recognized by the different monoclonal antibodies. Lymph nodes contain structures called high endothelial venules (HEV) which are utilized by lymphocytes to enter the lymph nodes (the site of immune responses) from the blood stream. Emigration of lymphocytes into the node has been shown to be mediated by adhesion molecules which allow the cells to stick to and then traverse the venule. This process has been studied by incubating isolated lymphocytes with lymph node tissue sections. When the sections are incubated with lymphocytes alone, the cells will adhere to HEV, and the number of adherent cells can be counted. Various monoclonal antibodies, including the LAM-1 antibodies, have been used to block this binding. Line 4 gives the results of such studies for LAM1-1, -2 and -3.

The polysaccharide PPME mimics the natural ligand for the LAM-1 molecule. Since PPME can be directly fluoresceinated, it is possible to study the effect of the various monoclonal antibodies on the interaction of LAM-1 and PPME. Line 5 details the results using cells which were first incubated with a LAM-1 monoclonal antibody, followed by treatment with PPME-FITC. Anti-TQl, anti-LAM1-2 and anti-LAM1-3 blocked PPME binding and anti-LAM1-1 enhanced PPME binding. These results demonstrate the functional (and by extrapolation, the specificity) differences between the antibodies.

The results shown on line 6 were obtained by the reverse of the line 5 experiment; i.e., cells were first incubated with unlabelled PPME, followed by indirect immunofluorescence with the LAM-1 antibodies. As in line 5, differences in the effects of incubation with PPME on subsequent monoclonal antibody binding indicate that the various monoclonal antibodies recognize different epitopes of the LAM-1 molecule.

Lines 7-10 detail the results of studies in which the ability of a given monoclonal antibody to block the subsequent binding of other monoclonal antibodies was analyzed. Blocking of one antibody by another provides evidence that the two antibodies in question recognize epitopes which are identical or close together on the molecule. The results in Table 1 indicate that anti-LAM1-1 does not block anti-LAM1-3, indicating that their epitopes are different. Anti-LAM1-2, however, does block anti-LAM1-3, indicating that these epitopes are at least close to each other. There are differences between anti-LAM1-2 and anti-LAM1-3, however, because of the difference in response they generate regarding Leu 8. Anti-LAM1-2 does not at all block Leu 8, whereas anti-LAM1-3 strongly blocks it. Species cross-reactivity gives further indications of the differences which exist between the antibodies and the epitopes that they identify.

Line 11 gives the results of the domain mapping regarding the monoclonal antibodies. The LAM-1 molecule contains three domains which are:
(a) a lectin-like domain (L);
(b) an epidermal growth factor-like domain (EGF); and
(c) a domain of short consensus repeats (SCR).
In order to determine which domain was recognized by each antibody, cDNAs were constructed which contained the information coding for:
(1) the whole LAM-1 molecule;
(2) the L, EGF and SCR domains from the LAM-1 molecule;
(3) the L domain from the LAM-1 plus EGF and SCR domains from the CD62 molecule of the same family of proteins;
(4) the L plus EGF domains from LAM-1 (SCR from CD62; and
(5) the L plus SCR domains from LAM-1 (EGF from CD62).
These cDNAs were transfected into cells which then produced the corresponding proteins. The pattern of reactivity of the various monoclonal antibodies was then determined as shown in Table 3, and the domain necessary for monoclonal antibody reactivity was assigned. For example, anti-LAM1-3 bound to cells expressing all the domains described with medium to very strong strength. Anti-LAM1-1, however, did not bind to cells which contained LAM-1 (L + SCR) or LAM-1 (L) alone. The epitope which is recognized by anti-LAM1-1 must, therefore, be composed of a site within the EGF domain, or which contains part of the L and EGF domains, but not the SCR domain. LAM1-3, on the other hand, must only contain the LAM-1(L) domain. The two antibodies are, therefore, distinguishable.

**Table 2.**

| Characteristics of Anti-LAM-1 Monoclonal Antibodies Line | | | | | |
|---|---|---|---|---|---|
| 1 | Antibody | TQ1 | LAM1-1 | LAM1-2 | LAM1-3 |
| 2 | Isotype ₁ | G1 ₇ | G1 ₇ | M | G1 ₇ |
| 3 | Fluoresc. | ++ | +++ | ++ | ++++ |
| 4 | HEV Bind² | N | B | wB | B |
| 5 | PPME Bind³ | B₇ | E | B | B |
| 6 | PPME Blocks ⁴ | - | +/- | +/- | - |

| Ability to Block Binding of Labelled Monoclonal Antibody | | | | | |
|---|---|---|---|---|---|
| 7 | TQ-1⁵ | +++ | - | +++ | +++ |
| 8 | Leu 8⁵ | - | ++ | - | +++ |
| 9 | LAM1-1⁵ | - | +++ | - | - |
| 10 | LAM1-3⁵ | +++ | - | +++ | +++ |
| 11 | Domain Map⁶ | L | L+EGF | L | L |

| Species Cross Reactivity | | | | | |
|---|---|---|---|---|---|
| 12 | Rhesus | - | +++ | - | +¹ |
| 13 | Tamarin | - | +++ | ++ | -- |
| 14 | Cow | ND | ND | ND | +++ |
| 15 | Rabbit | +++ | - | ++ | ++++ |
| 16 | Sheep | - | - | - | +++ |
| 17 | Dog | - | - | - | +++ |
| 18 | Cat | - | - | - | +++ |
| 19 | Pig | ND | ND | ND | + |
| 20 | Goat | ND | ND | ND | +++ |
| 21 | Epitope | C | A | B | D |

| | | | | | |
|---|---|---|---|---|---|
| 1 = Fluorescence intensity, human lymphocytes and peripheral mononuclear cells. Fluorescence intensity of staining in indirect immunofluorescence assays is given on a 4-(+) scale where (-) indicates no specific reactivity and (++++) indicates the highest level of activity. | | | | | |
| 2 = Monoclonal antibody blocking, High Endothelial Venule binding. | | | | | |
| 3 = Monoclonal antibody blocking, phosphomannan monoester fragments (PPME). | | | | | |
| 4 = PPME blocking monoclonal antibody. | | | | | |
| 5 = Ability to block binding of antibody. | | | | | |
| 6 = Domain mapped. | | | | | |

### Abbreviations

- Bind =: Binding
- B =: Blocks
- wB =: weakly Blocks
- E =: Enhances
- ND =: Not Done
- L =: Lectin
- EGF: = Epidermal Growth Factor-like
- SCR: = Short Consensus Repeats
- N =: No effect

**Table 3.**

| Structural domains identified by the anti-LAM-1 monoclonal antibody reactive with fusion proteins a containing LAM-1 domains ^{a} | | | | | |
|---|---|---|---|---|---|
| Test mAb Lectin | Whole | Lectin | Lectin | Lectin | |
| | LAM-1 | | EGF | EGF SCRs | SCRs |
| anti-LAM1-1 | +++ | - | +++ | +++ | - |
| anti-LAM1-2 | ND | ND | ND | ND | ND |
| anti-LAM1-3 | +++ | +++ | ++ | ++++ | ++ |

| | | | | | |
|---|---|---|---|---|---|
| a = Values represent the relative intensity of immunofluorescence staining of COS-7 cells transfected with the LAM-1 cDNA or recombinant cDNAs encoding the lectin, EGF-like, or two SCR domains of LAM-1 with the rest of the cDNAs encoding CD62. | | | | | |
| SCR = a domain of short consensus repeats. | | | | | |
| EGF = epidermal growth factor-like domain. | | | | | |

## Claims

1. A hybridoma cell line which has American Type Culture Collection Deposit No. HB 10771.

2. An anti-LAM1-3 monoclonal antibody obtainable from the hybridoma cell line according to claim 1.

3. A chimeric antibody comprising the variable region of the anti-LAM1-3 antibody according to claim 2 and a constant region derived from a human monoclonal antibody.

4. An antibody according to claim 2 or claim 3, for use in therapy.

5. An antibody according to claim 2 or claim 3, for use in blocking adhesion, migration and infiltration into tissues of cells expressing LAM-1.

## Patentansprüche

1. Hybridom-Zelllinie mit der ATCC-Hinterlegungsnummer HB 10771.

2. Monoclonaler anti-LAM1-3-Antikörper erhältlich von der Hybridom-Zelllinie nach Anspruch 1.

3. Chimärer Antikörper, umfassend die variable Region des anti-LAM1-3-Antikörpers nach Anspruch 2 und eine konstante Region, die aus einem menschlichen monoclonalen Antikörper stammt.

4. Antikörper nach Anspruch 2 oder 3 zur Verwendung in der Therapie.

5. Antikörper nach Anspruch 2 oder 3 zur Verwendung für die Verhinderung von Adhäsion, Migration und Infiltration in die Gewebe von LAM1 exprimierenden Zellen.

## Revendications

1. Lignées de cellules d'hybridome qui a un No. de dépôt au American Type Culture Collection HB 10771.

2. Anticorps monoclonal anti-LAM1-3 pouvant être obtenu de la lignée de cellules d'hybridome selon la revendication 1.

3. Anticorps chimérique comprenant la région variable de l'anticorps anti-LAM1-3 selon la revendication 2 et une région constante dérivée d'un anticorps monoclonal humain.

4. Anticorps selon la revendication 2 ou la revendication 3, à utiliser en thérapie.

5. Anticorps selon la revendication 2 ou la revendication 3, à utiliser dans le blocage de l'adhérence, de la migration et de l'infiltration dans le tissus des cellules exprimant LAM-1.
